Europäisches Patentamt

European Patent Office

Office européen des brevets

(11) Publication number: 0 275 606
A1

# EUROPEAN PATENT APPLICATION

(21) Application number: 87202610.9

(22) Date of filing: 22.12.87

(51) Int. Cl.⁴: C12N 15/00 , C12N 9/72 , A61K 37/54

(30) Priority: 06.01.87 NL 8700013

(43) Date of publication of application:
27.07.88 Bulletin 88/30

(84) Designated Contracting States:
AT BE CH DE ES FR GB GR IT LI LU NL SE

(71) Applicant: Leuven Research & Development
V.Z.W.
Benedenstraat 59A Groot Begijnhof
B-3000 Leuven(BE)

Applicant: Collen, Désiré José
Schoonzichtlaan 20
B-3009 Winksele-Herent(BE)

(72) Inventor: Collen, Désiré José
Schoonzichtlaan 20
B-3009 Winksele-Herent(BE)
Inventor: Holmes, William Evans
Bovenstraat 87/3 Groot Begijnhof
B-3009 Winksele-Herent(BE)
Inventor: Nelles, Lucien Georges Raymond
Geerdegemdries 21
B-2800 Mechelen(BE)
Inventor: Lijnen, Henri Roger
Acacialaan 50A
B-3020 Herent(BE)

(74) Representative: Prins, Hendrik Willem et al
Octrooibureau Arnold & Siedsma
Sweelinckplein, 1
NL-2517 GK The Hague(NL)

(54) Hybrid plasminogen activators with improved thrombolytic properties and drugs comprising these plasminogen activators.

㊐ The invention relates to plasminogen activators obtained by expression of fused cDNA encoding fragments of human tissue-type plasminogen activator (t-PA) and of human urokinase-type plasminogen activator (u-PA), by combining the fibrin-affinity of t-PA with the fibrin-specificity of u-PA in the single chain form (scu-PA) and/or with the enzymatic activity of u-PA having the two-chain form (tcu-PA). It is preferably a hybrid molecule obtained by fusion of at least a region part of the NH₂-terminal region of t-PA to at least a part region of the COOH-terminal region of scu-PA, most preferably the NH₂-terminal amino acids 1 to 263 of t-PA, fused to the COOH-terminal amino acids 144 to 411 of scu-PA.

The invention relates further to a recombinant DNA molecule which yields said hybrid plasminogen activators; the respective mammalian expression vector containing said DNA sequence; a transfected mammalian cell line which comprises said recombinant DNA molecule; and to the method for the preparation of plasminogen activators.

## Hybrid plasminogen activators with improved thrombolytic properties and drugs comprising these plasminogen activators

The invention relates to new plasminogen activators which are hybrids of plasminogen activators of the tissue-type (t-PA) and of the plasminogen activator of the urokinase-type with the one-chain form, which hybrids have improved thrombolytic properties. This invention relates further to a method of the preparation of these new plasminogen activators, and to drugs comprising these plasminogen activators.

It is known that certain enzymes, called plasminogen activators, after intravenous infusion in man or in animals can exert a thrombolytic action in vivo. This action is based on activation of plasminogen present in blood, which initiates a chain of reactions resulting in the lysis of a fibrin-containing blood clot in the blood vessels. The mechanisms of action are complex and can be different depending on the type of plasminogen activator which is used.

Plasminogen activators can be differentiated into two types, i.e., plasminogen activator of the tissue-type (t-PA) and plasminogen activator of the urokinase-type (u-PA). The plasminogen activators of the urokinase-type can be subdivided into a two-chain form (tcu-PA) and a one-chain form (scu-PA).

t-PA can cause activation of plasminogen but is much more efficiently in the presence of fibrin than in its absence. This means that activation mainly occurs in the vicinity of a blood clot, which is dissolved, while much less activation of plasminogen occurs in circulating blood. t-PA does not react with antisera against tcu-PA or scu-PA.

tcu-PA induces the activation of plasminogen in blood both in presence and absence of fibrin. This means that blood clots can be dissolved, but also that activation of the fibrinolytic system in circulating blood occurs, which leads to fibrinogen breakdown and a bleeding tendency.

scu-PA activates plasminogen efficiently, but only in the presence of fibrin-containing blood clots. Likewise as with t-PA little activation of the fibrinolytic system in circulating blood will occur, notwithstanding the fact that the mechanism of activation is different and the fact that scu-PA is immunologically unrelated to t-PA. Of these three plasminogen activators tcu-PA has been used in medical practice since a long time, during which the described side effects have been a handicap.

Although many questions relating to the optimal use of these active compounds remain unanswered hitherto, it has been established that their fibrin-specificity is not as pronounced in man as was anticipated from several animal models. Therefore, the quest for thrombolytic agent with a higher specific thrombolytic activity and a better fibrin-selectivity remains open.

One approach to the development of improved thrombolytic agents consists of the production of hybrid proteins which contain the structures which are responsible for the fibrin-specificity of both t-PA and scu-PA.

The fibrin-specificity of t-PA is mainly due to its affinity for fibrin and the remarkable enhanced activity of plasminogen at the fibrin surface. The structures in t-PA responsible for its fibrin-activity are localized its NH$_2$-terminal region of the molecule, probably in the finger-like domain and in the second kringle. u-PA only displays fibrin-specificity in the scu-PA form, in which the Lys$^{158}$-Ile$^{159}$ peptide bond is intact. The fibrin-specificity is not dependent on the presence of the terminal 143 amino acids nor on the potential cleavage of the Lys$^{158}$-Lys$^{159}$ peptide bond. Thus hybrid proteins consisting of the NH$_2$-terminal region of t-PA and of the COOH-terminal region of scu-PA and in which the conversion to u-PA is abolished, might have a higher fibrin-selectivity than t-PA or scu-PA and thereby constitute better thrombolytic agents.

On the basis of this hypothesis, hybrid molecules of t-PA and u-PA have already been constructed by transient expression of cDNA fragments of t-PA (encoding amino acids 1 to 67, 1 to 212 or 1 to 313) fused to cDNA fragments of scu-PA (encoding amino acids 136 to 411, 139 to 411 or 195 to 411 respectively). These recombinant products, following cleavage by plasmin, activate plasminogen at a very similar rate as u-PA, which indicates that fusion of t-PA structures to u-PA does not impair the catalytic activity of the enzyme. Further investigations with one of these hybride molecules (1 to 67 of t-PA with 136 to 411 of scu-PA) however revealed that it had not acquired the fibrin-activity of t-PA nor an improved fibrin-selectivity as compared to scu-PA.

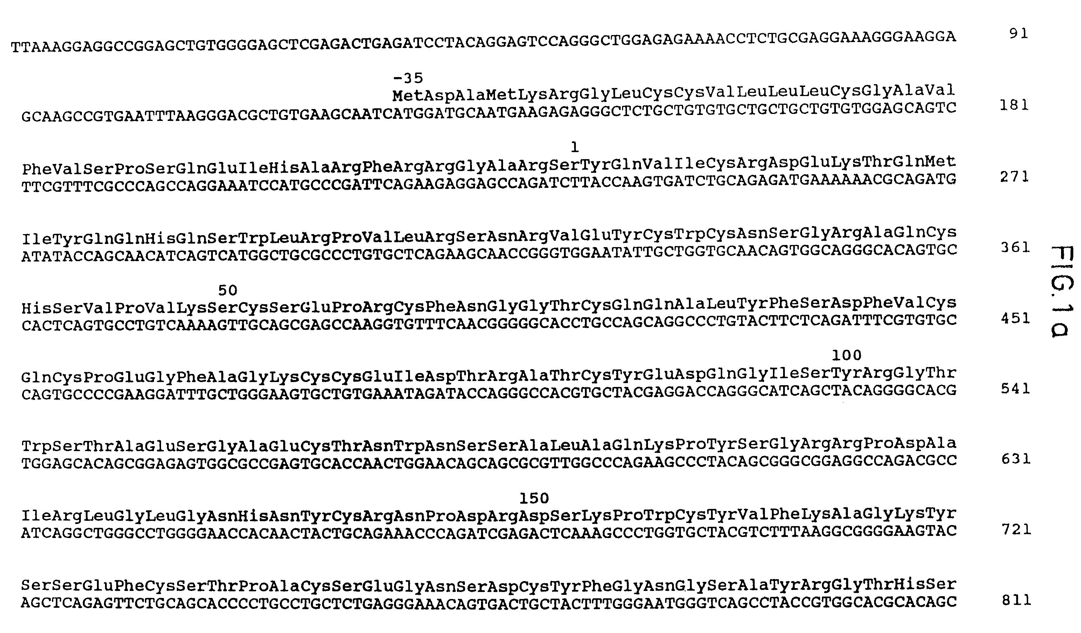

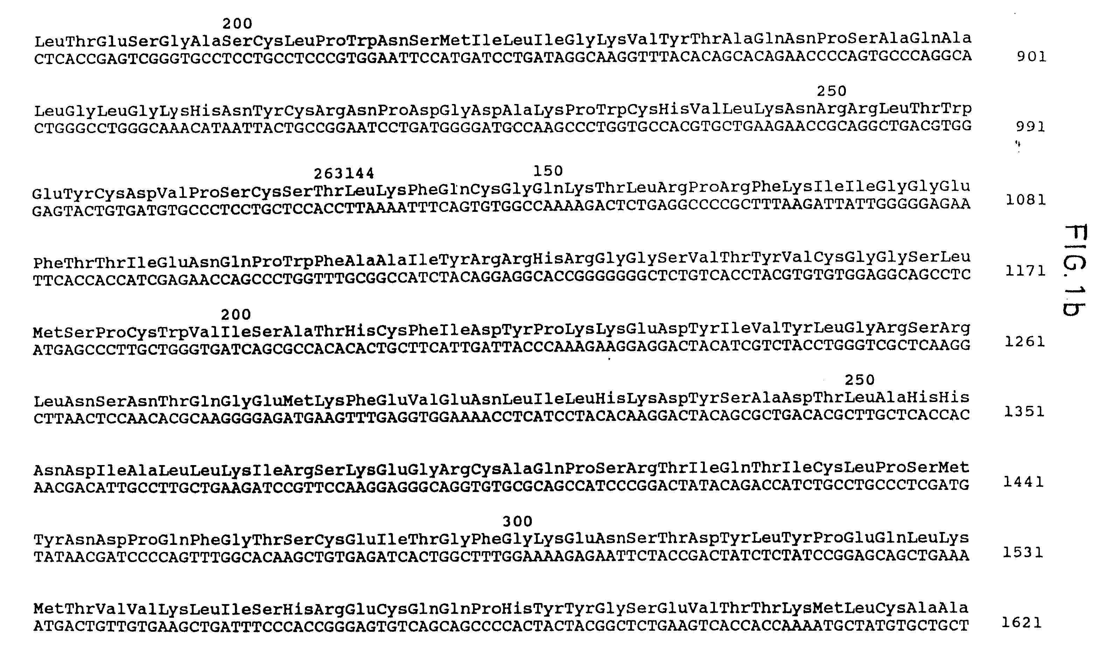

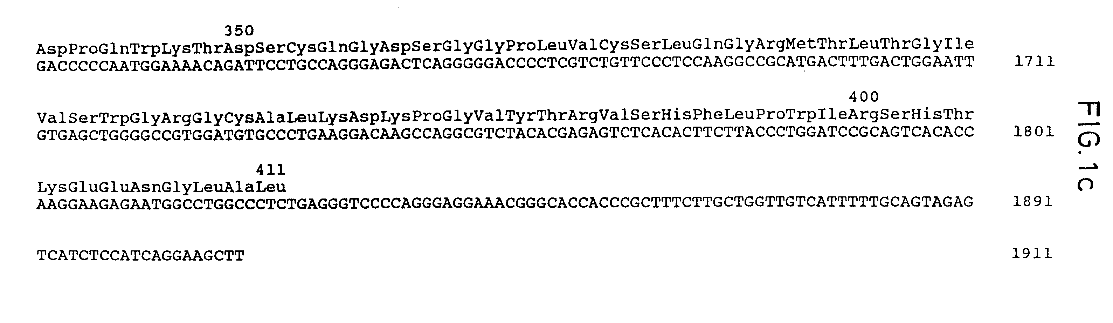

Continued research resulted in a hybrid molecule that expresses the combined mechanisms of fibrin-specificity of t-PA and activity of scu-PA. It is the very first time that a hybrid molecule is provided in which an improved fibrine-selectivity and a more specific thrombolytic activity are concomitantly expressed. In a preferred embodiment the hybrid molecule consists of the NH$_2$-terminal amino acids 1 to 263 of t-PA and in frame therewith the COOH-terminal amino acids 144 to 411 of scu-PA (fig. 1). In addition we found that in a plasma milieu in vitro this hybrid molecule has a higher specific thrombolytic activity and a better fibrin-specificity in comparison with scu-PA. Further similar hybrid molecules possess a fibrinolytic action on fibrin-containing intergrowths on exudation mass. Benefitial action is also expected with atonal wounds caused by a subnormal bloodcirculation.

With respect to the NH₂-terminal amino acids of t-PA it is noted that at least the amino acids of the finger (amino acids 1 to 44 or 45) and that of the second kringle (amino acids 178 to 261 or 262) must be present. Optionally two kringles in tandem may be present. Among the COOH-terminal amino acids of scu-PA at least the amino acids starting from amino acid 148 must be present. The peptide chain may be increased up to preferably amino acid 144 or even amino acid 136.

The invention relates to methods for the production of these new hybrid plasminogen activators having improved fibrin-specificity, methods for the construction of chimaeric cDNA molecules encoding such hybrid plasminogen activators, for the mammalian cell systems in which these cDNA molecules can be expressed, for methods for the purification of culture media and extracts of these cell systems, and for pharmaceutical compositions and preparation methods for the treatment of patients with thrombolytic diseases.

In the preparation of a pharmaceutical composition the hybrid plasminogen activator may be combined with any pharmaceutically acceptable exipient suitable for the stated use. Further it is possible to incorporate next to these hybrid plasminogen activators other plasminogen activators, whereby synergetic action may occur. Preferably the pharmaceutical composition has the form of an intravenous infusion liquid since it is this form which results in superior results.

The invention is further illustrated in the following experiments without being limited thereto.

## I Construction of t-PA cDNA clones

t-PA was purified from a conditioned medium of melanoma cells (Bowes). Total RNA was isolated from Bowes human melanoma cells with the known guanidinium thiocyanate extraction/lithiumchloride precipitation method. Poly A mRNA was prepared by oligo-dT chromatography. Oligo-dT-primed cDNA was prepared according to a known method and cloned in λgt11 (T.V. Huynn et al, λgt10 and λgt11 DNA cloning techniques: a practical approach, IRC Press, Oxford (1984)).

Two partially overlapping deoxyoligonucleotides representing complementary strands of t-PA cDNA were chemically synthesized. These two strands 5'-GGGCACCTGCCAGCAGGCCCTGTACTTCTC-3' and 5'-GGGCACTGGCACACGAAATCTGAGAAGTAC-3' were annealed and filled in using the Klenow fragment of E. coli DNA polymerase I (final concentration 0.1 Units/ml, Boehringer Mannheim), and 200μM each of dGTP, dTTP and dATP (Pharmacia, Uppsala, Sweden) and 250μCi (α-$^{32}$P) dCTP, 5,000 Ci mmole (New England Nuclear, Boston, MA) to produce a 49 nucleotide long probe with a specific acitvity of ~$10^8$ cpm μg. This probe spans nucleotides 366 through 416 of the cDNA sequence (D. Pennica et al, Nature 301, 214-221 (1983)). Plaque hybridization was performed with this probe.

cDNA from positive plaques (λt-PA4 and λt-PA8) were spliced together with pUC18 to produce a contiguous cDNA sequence encoding t-PA. t-PA cDNA sequences were then subcloned into M13mp18 or M13mp19 (C. Yannish-Perron et al, Gene 33, 103-118, (1985)), and sequenced by the dideoxynucleotide chain termination method.

## II Construction of u-PA cDNA clones

pUCscu-PA containing cDNA sequences encoding full length scu-PA were obtained as follows. Total RNA was isolated from CALU-3 cells (ATCC, HTB-55) by the guanidinium isothiocyanate extraction/lithium chloride precipitation method. Poly A mRNA was prepared by oligo-dT cellulose chromatography. Oligo-dT-primed cDNA was prepared and cloned into λgt11.

Two partially overlapping deoxyoligonucleotide representing complementary strands of scu-PA cDNA were chemically synthesized. These two strands 5'-GGGCAGGTGTGCGCAGCCATCCCGGACT and 5'-GGGCAGGCAGATGGTCTGTATAGTCCGGG were annealed and filled in using the Klenow fragment of E. coli DNA polymerase I (final concentration 0.1 unit/μl) and 200μM dGTP, dTTP and dATP, and 250μCi (α-$^{32}$P) dCTP to produce a 49 nucleotide long probe having a specific activity of ~$10^8$ cpm μg. This probe spans nucleotides 931 through 979 of the cDNA sequence (W.E. Holmes et al, Biotechnology 3, 923-929, (1985)). Then plaque hybridization was performed. A second specifically primed cDNA library was prepared in λgt11, using the latter mentioned deoxyoligonucleotide to initiate first strand cDNA synthesis. Three overlapping cDNA clones were spliced together to produce a contiguous cDNA sequence encoding full length scu-PA.

## III Construction of t-PA/u-PA hybrid cDNA clones

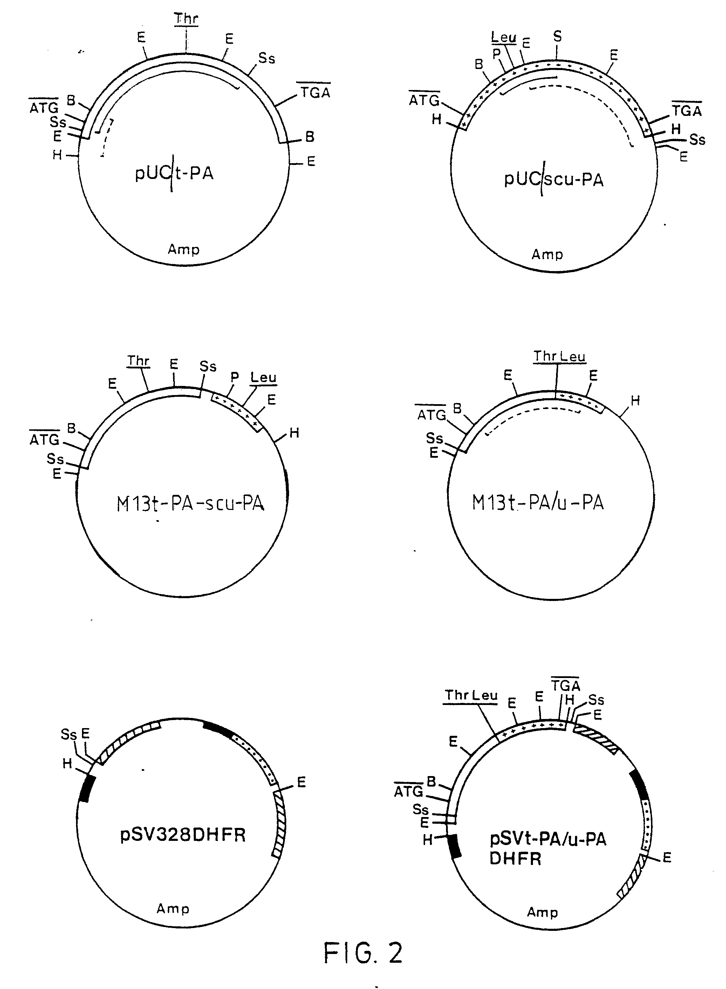

The BglII-Sau3AI fragment from pUCscu-PA (nucleotides 399 to 732, encoding amino acids Arg[88] through Val[39]) was ligated into BamHI digested M13mp18 to yield M13intl. The SstI fragment of pUCt-PA (nucleotides -20 to 1422) encoding the amino acids from the terminal Met[35] through Leu[41] was then ligated into the SstI site of M13intl, upstream of the scu-PA sequence to yield M13t-PA scu-PA (see fig. 2). Both t-PA and scu-PA sequences are in the same orientation in this DNA molecule. Splicing of the DNA at t-PA codon Thr[263] and scu-PA codon Leu[44] was accomplished by in vitro site directed mutagenesis using the single stranded template DNA of M13t-PA-scu-PA and the synthetic deoxyoligonucleotide deletion primer 5'-CTGAAATTTTAAGGTGGAGCAGGA-3' (New England Biolabs), which is complementary to the codons for amino acids Ser[262] to Thr[263] of t-PA and for the amino acids Leu[44] to Gln[47] (Thr[263]) of scu-PA. 200ng of the phosphorylated deoxyoligonucleotide was heated with 1μg of the template at 65°C in 50mM Tris-HCl buffer pH 7.8, 10mM $MgCl_2$ and 20mM DTT for 5 minutes and annealed by stepwise cooling to room temperature and 0°C. ATP was added to a concentration of 0.4mM and dNTPS to a concentration of 0.05mM in a final volume of 50μl. 400 U $T_4$ DNA ligase and 5 units Klenow fragment of E. coli DNA polymerase I were added. The DNA obtained after 1 hr incubation at 14°C was used to transfect E. coli JM 101 cells.

250ng of the deletion primer was radiolabeled to a specific activity of $10^8$ cpm/μg by incubation inn 70mM Tris-HCl buffer, pH 8.0, containing 10mM $MgCl_2$ and 5mM DTT, of 100μCi of ($\alpha$-$^{32}$P)ATP and 10 units of $T_4$-polynucleotide kinase in a 20μl reaction mixture, for 30 minutes at 37°C. This labeled probe was then used for plaque hybridization to identify the spliced DNA of M13t-PA/u-PA. Hybridization proceeded overnight at 22°C in 0.09M Tris-HCl buffer pH 7.5, containing 0.9M NaCl, 0.5% Nonidet P40 detergent, 1$^\times$ Denhardts, 6mM EDTA, 1mM Na-pyrophosphate, 0.1mM ATP and 0.2 mg/ml E. coli tRNA. Nitrocellulose filters were washed for 1 hr in 75mM NaCl at 49°C with several changes and exposed overnight to X-ray films. Plaques revealing a positive hybridization signal were selected for dideoxynucleotide sequencing in order to verify the deletion of undesired DNA.

## IV Eukaryotic expression of t-PA/u-PA hybrid cDNA clones

The final expression plasmid was constructed in two steps. The BglII-partial EcoRI restriction endonuclease fragment from M13t-PA/u-PA encoding Ser[1] through Thr[263] in the t-PA sequence and Leu[44] through Glu[163] of the scu-PA sequences and the partial EcoRI-SstI restriction endonuclease fragment of pUCscu-PA encoding Phe[164] through Leu[41], 78 nucleotides of 3'-untranslated sequence of scu-PA cDNA and a remnant of pUC18 polylinker, were ligated into BglII-SstI digested pUCscu-PA

The HindIII-BglII restriction endonuclease fragment of pUCt-PA containing 5'-untranslated sequences of t-PA cDNA and encoding t-PA amino acids Met[35] through Arg`, the BglII-SstI restriction endonuclease fragment of pInt1 (hybrid amino acids Ser` through Leu[531] and 78 nucleotides of 3'-untranslated sequence) and the SstI-HindIII vector fragment of pSV328DHFR were ligated to yield pSVt-PA/u-PADHFR (fig. 2).

5μg of the pSVt-PA/u-PADHFR plasmid were used to transfect DHFR deficient Chinese Hamster Ovary cells. These cells were obtained from W. Fiers of the University of Gent, Belgium. The cells were transfected using the known calcium phosphate coprecipitation method. DHFR+ cells were isolated and were monitored for secrection of low molecular weight u-PA related antigen using an ELISA assay.

Large scale production was carried out, in which conditioned medium was harvested three times after consecutive 2 day incubation period in serum free medium.

## V Purification of t-PA/u-PA hybrid proteins from conditioned cell culture media

The hybrid proteins were purified by chromatography on Zinc chelate-Sepharose and immunoadsorption on an insolubilized murine monoclonal antibody (MA-1C8) which abolishes the binding of t-PA to fibrin. Approximately 15 liters of conditioned media were applied to a 5$^\times$ 25 cm column of Zinc chelate-Sepharose, previously equilibrated with 0.3M NaCl, 0.02M Tris-HCl buffer, pH 7.5, containing 0.01% Tween 80 and 10 KIU ml aprotinin, at a flow rate of 250ml per hr at 4°C. The column was washed with equilibration buffer and eluted with buffer containing 50mM imidazole. Fractions of 7.5 ml were collected into tubes containing 2.5 ml 0.4M arginine, 0.08M citrate buffer pH 5.0, and 40 KIU ml of aprotinin. The u-PA related antigen as measured by ELISA, eluted in a peak which virtually coincided with the main protein peak.

Half of the pooled u-PA containing fractions of the Zinc chelate column were chromatographed on a 0.9 7 cm immunoadsorption column at a flow rate of 8 ml per hr. The column was washed with 0.3M NaCl, 0.02M Tris-HCl buffer pH 7.5, containing 0.01% Tween 80 and 10 KIU/ml aprotinin and then eluted with 2M KSCN in the same buffer. Fractions of 7.5ml were collected into tubes containing 2.5ml 0.4M arginine, 0.08M citrate buffer, pH 5.0, and 40 KIU/ml of aprotinin. These fractions containing u-PA related antigen were pooled and dialyzed against 0.3M NaCl-0.1M arginine-0.02M citrate buffer, pH 5.0 containing 0.01% Tween 80 and 10 KIU/ml of aprotinin.

This purification step with Zinc chelate-Sepharose had a recovery of 85% and a reduction in volume of 75 fold. Immunoadsorption chromatography yielded 3.0mg u-PA related antigen in a total volume of 10ml. This purification step gave a purification factor of 24 fold and a 10 fold reduction in volume. The overall yield of u-PA related antigen was 53% and 420µg purified material were obtained per liter conditioned medium. When aprotinin was added to a concentration of 10 KIU/ml to the cell culture media and the buffers were used during purification, t-PA/u-PA hybrid protein was obtained nearly exclusively in the t-PA/scu-PA form.

## VI Characterization of the t-PA/scu-PA and t-PA/tcu-PA hybrids

The purified t-PA/scu-PA hybrid migrated as a doublet band under non-reducing conditions on SDS-PAGE, but as a single main band with approximately $M_r$ 70,000 under reducing conditions. Both bands of the doublet seen on the unreduced gel are both immunologically active with antisera against u-PA and antisera against t-PA.

The amino acid composition of the t-PA/scu-PA hybrid is represented in table I. The observed amino acid compositions are in agreement with those derived from the known sequences of the t-PA/scu-PA hybrid.

The specific acitivity on fibrin plates was 175,000 IU/mg for the t-PA/scu-PA hybrid and for the t-PA/tcu-PA hybrid 205,000 IU/mg, as compared to 160,000 IU/mg for scu-PA and 50,000 IU/mg for t-PA.

### VI.1 Treatment with plasmin

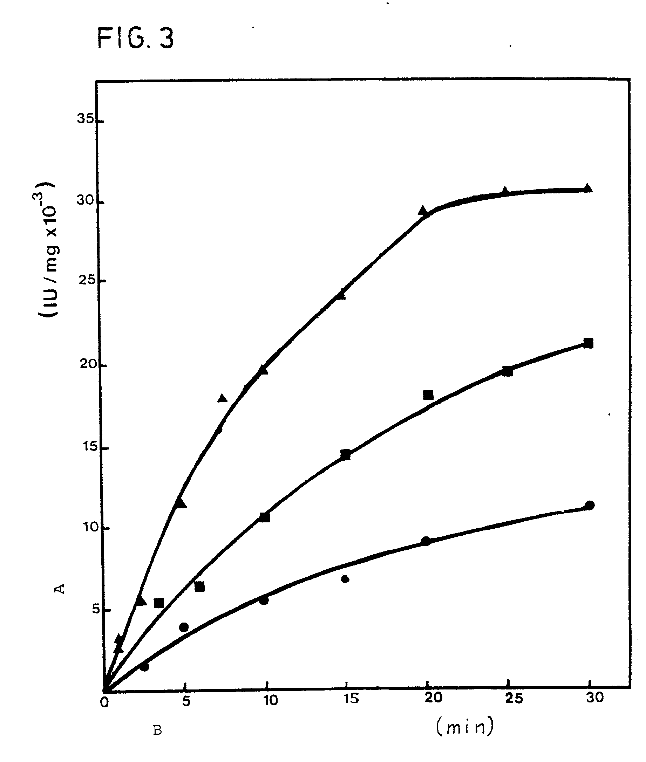

The t-PA/scu-PA hybrid solution in 0.02M citrate buffer, pH 5.0, containing 0.3M NaCl, 0.2M arginine and 0.01% Tween 80 was diluted 3-fold in 0.038M NaCl, 0.05 M Tris-HCl buffer, pH 8.4, containing 0.01% Tween 80 (final concentration 2.0µM), and treated with plasmin (final concentration 30-90nM) at 37°C. At timed intervals (0 to 30 minutes), 5µl were removed and added to 800µl of the same buffer containing S-2444 at a final concentration of 0.3mM. Urokinase activity was determined from measurements of the absorption at 405nm and expressed in IU by comparison with the International Reference Preparation (66/46). The molecular form of the hybrid at the end of the treatment was monitored on 12% SDS-PAGE after reduction. The specific activity of the t-PA/scu-PA hybrid was 340 IU/mg. Plasmin caused a time-dependent conversion of the t-PA/scu-PA hybrid to amidolytically active urokinase (see fig. 3: A = specific amidolytic activity, B = time in minutes). Maximal conversion results in an amidolytic activity corresponding to a specific activity of about 33,000 IU/mg for the t-PA/scu-PA hybrid. No effect of 0.1M arginine was observed on hydrolysis of S-2444 by urokinase.

SDS-PAGE shows complete conversion of the t-PA/scu-PA hybrid from a one-chain form to a two-chain molecule following incubation with plasmin (3% on molar basis) for 30 minutes at 37°C.

### VI.2 Activation of plasminogen by t-PA/scu-PA hybrid

The activation of plasminogen by the t-PA/scu-PA hybrid was measured in the presence of excess substrate. The t-PA/scu-PA hybrid at a concentration of 25nM was incubated with varying concentrations of plasminogen (final concentration 0.25 to 2µM) at 37°C in 0.038M NaCl, 0.05M Tris-HCl buffer, pH 7.4, containing 0.01% Tween 80 and 1mM S-2251. The generation of plasmin was determined from the absorption at 405nm, monitored over 3 to 4 minutes and expressed in nM by comparison with a plasmin calibration curve.

Activation of plasminogen (final concentration 10 to 95µM) by the t-PA/scu-PA hybrid (final concentration 25nM) was measured by incubation of plasminogen at 37°C in 0.05M Tris-HCl buffer pH 7.4 containing 0.038M NaCl and 0.01% Tween 80. The generated plasmin at different time intervals (0 to 6 minutes) was measured with S-2251 (final concentration 0.3mM) after 200-fold dilution of the sample. Initial activation

rates were obtained from plots of the concentration of generated plasmin versus incubation time.

The effect of fibrin on the activation rate of plasminogen by the t-PA/tcu-PA hybrid was evaluated by incubation of plasminogen (final concentration 1.5μM) at 37°C in 0.05M Tris-HCl buffer pH 7.4 containing 0.038M NaCl and 0.01% Tween 80 with 0.25 or 0.50μM CNBr-digested fibrinogen prior to addition of t-PA/tcu-PA (final concentration 1.25nM). The plasmin generated was measured following 20-fold dilution of the sample.

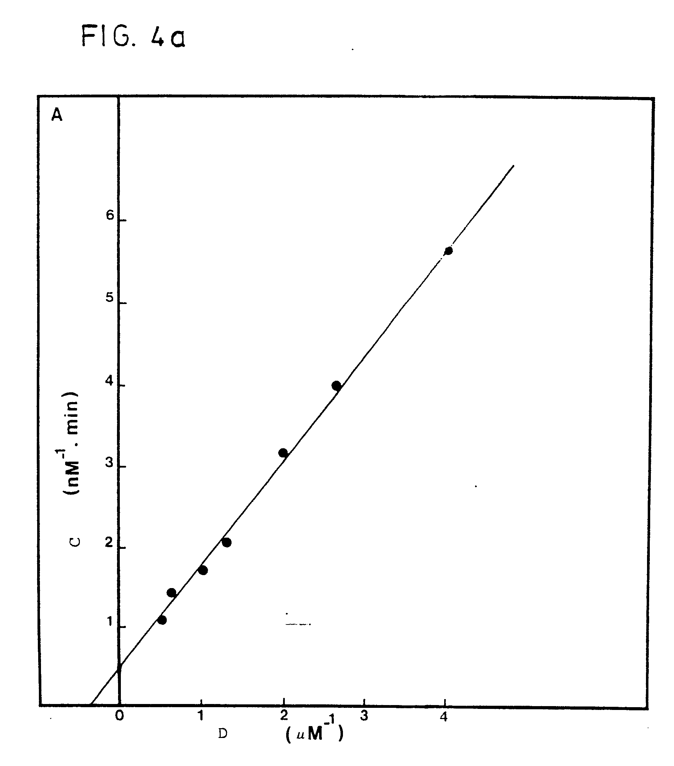

Activation of plasminogen by t-PA/scu-PA hybrid obeys Michaelis-Menten kinetics (see fig. 4A: C = the reciproque of the initial activation rate (v) and D = the reciproque of the plasminogen activation concentration). No effect of 0.1M arginine was observed on the activation rate of plasminogen or by the hydrolysis rate of S-2251 by plasmin. From these Lineweaver-Burk plots, $K_m = 2.2\mu M$ and $k_2 = 0.0013$ s' were obtained (r = 0.998).

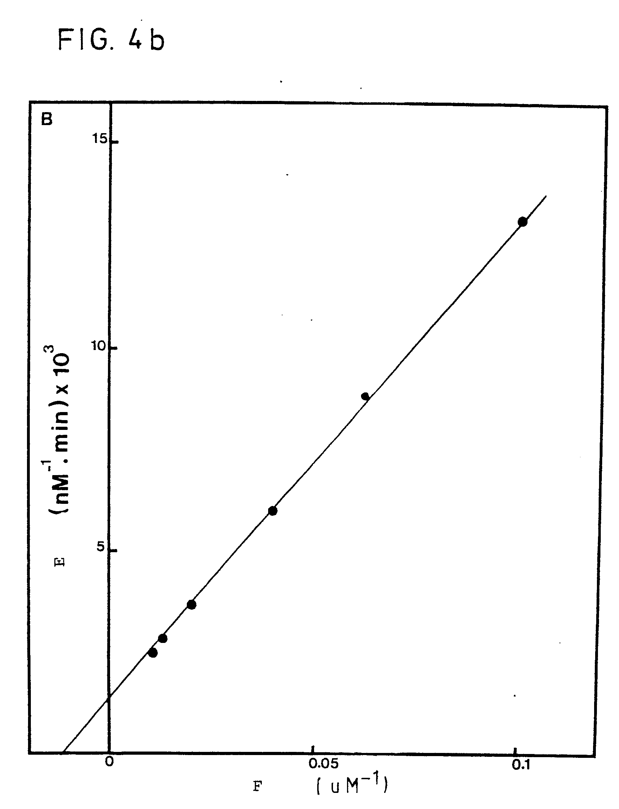

Activation of plasminogen by t-PA/tcu-PA hybrid was obeyed by Michaelis-Menten kinetics (fig. 4B in which E and F corresponds to C and D from fig. 4A). $K_m = 85\mu M$ and $k_2 = 5s$' (r = 0.999).

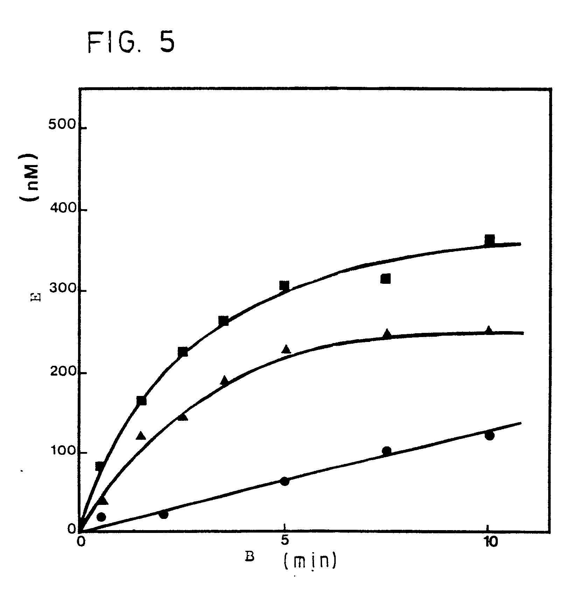

Addition of CNBr-digested fibrinogen to the incubation mixture of plasminogen and the t-PA/tcu-PA hybrid resulted in a marked enhancement of the activation rate (see fig. 5; E = plasmine concentration, B = time).

## VI.3 Binding to purified fibrin

Human fibrinogen (final concentration 0 to 3.3 mg/ml) in 0.038M NaCl, 0.05M Tris-HCl buffer pH 7.4, containing 0.01% Tween 80 and 1 mg.ml BSA, was mixed with scu-PA, tcu-PA, t-PA/scu-PA, t-PA/tcu-PA or t-PA (final concentration 50 to 100 ng/ml). The mixture was clotted by addition of thrombin to a final concentration of 20 NIH units/ml. Following one minute incubation at 37°C, thrombin was inactivated by the addition of D-Ile-Pro-Arg-CH₂Cl (10μM final concentration) to prevent inactivation of urokinase and the samples were centrifuged for one minute at 10,000g. The concentrations of u-PA or t-PA related antigen in the supernatants were determined by the afore-mentiond ELISA assay.

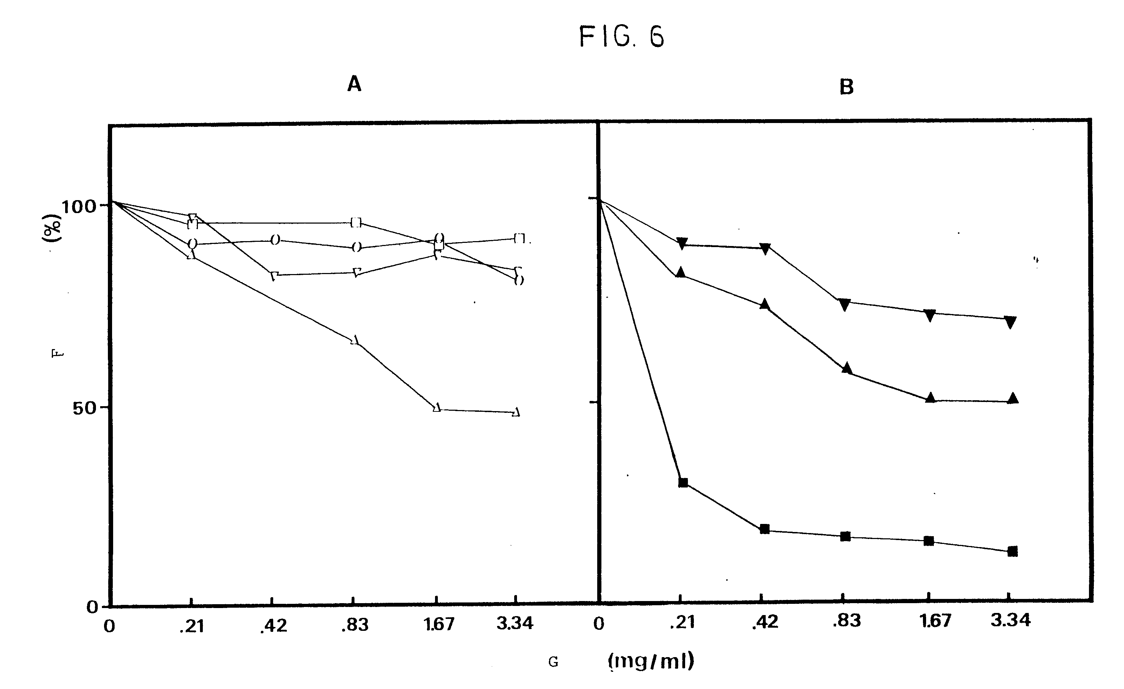

Fig. 6 (F = remaining antigen, G = fibrin) illustrates that t-PA, the t-PA/scu-PA hybrid and to a lesser extend the t-PA/tcu-PA hybrid have a specific affinity for fibrin, which is not displayed by scu-PA or tcu-PA

## VI.4 ¹²⁵I-fibrin labeled plasma clot lysis in a plasma milieu

The relative fibrin specificity of scu-PA, tcu-PA, t-PA/scu-PA, t-PA/tcu-PA and t-PA was measured in a system composed of a ¹²⁵I-fibrin labeled human plasma clot, suspended in human citrated plasma (C. Zamarron et al. Thromb. Haemost. 52, 19-23, 1984). Fibrinogen levels were measured with a coagulation rate assay. The relative stability of the plasminogen activators in plasma was measured by addition of the plasma clot after 4 hours preincubation of the enzymes in plasma at 37°C. Both releases of radioisotope and fibrinogen breakdown were monitored.

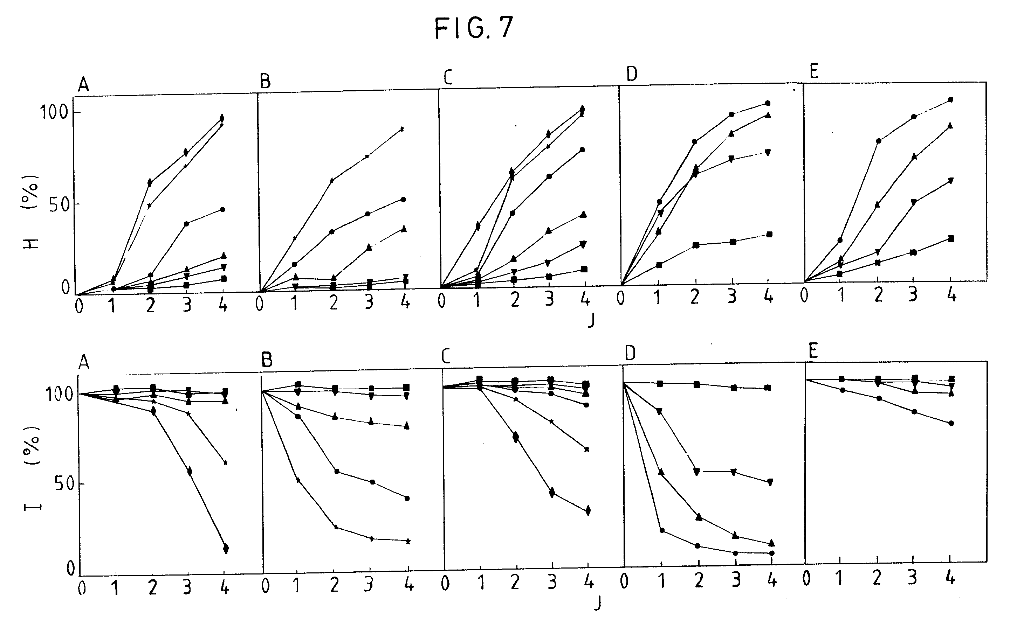

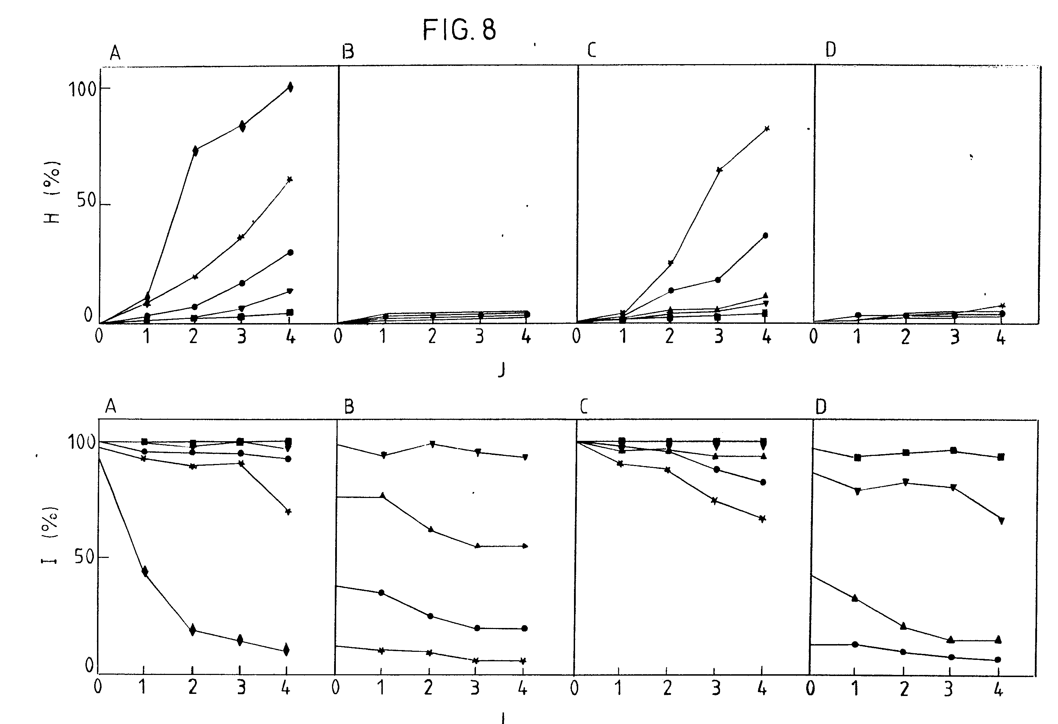

All plasminogen activators tested induced a concentration dependent lysis of a ¹²⁵I-fibrin labeled clot immersed in human plasma (fig. 7: H = lysis, I = remaining fibrinogen, J = time in hours). Clot lysis was associated with a high degree of fibrinogen breakdown for t-PA and the t-PA/tcu-PA hybrid. The t-PA/scu-PA hybrid had a significantly higher specific thrombolytic activity than scu-PA. Preincubation of the plasminogen activators with plasma resulted in inhibition of the fibrinolytic potential of t-PA and the t-PA/tcu-PA hybrid but not of scu-PA or the t-PA/scu-PA hybrid. The preincubation caused fibrinogenolysis comparable to that obtained in the presence of the clot (see fig. 8 in which H, I and J have the same meaning as in fig. 7).

List of abbreviations
$M_r$ : molecular mass
u-PA : urokinase-type plasminogen activator
tcu-PA : two-chain u-PA
scu-PA : single chain u-PA
rscu-PA : recombinant scu-PA obtained by expression of cDNA in a mammalian cell system
nscu-PA : natural scu-PA purified from human lung adenocarcinoma cell (CALU-3) conditioned medium
t-PA : tissue-type plasminogen activator
rt-PA : recombinant t-PA obtained by expression of cDNA in a mammalian cell system
nt-PA : natural t-PA purified from human melanoma (Bowes) cell conditioned medium

t-PA.u-PA : recombinant fusion protein containing amino acids Ser¹ through Thr²⁶³ of t-PA and amino acids Leu¹⁴ through Leu⁴¹¹ of u-PA, with the Lys¹⁵⁸-Ile¹⁵⁹ peptide bond either intact or cleaved

t-PA.scu-PA : t-PA.u-PA with the Lys¹⁵⁸-Ile¹⁵⁹ peptide bond intact t-PA.tcu-PA : t-PA.u-PA with the Lys¹⁵⁸-Ile¹⁵⁹ peptide bond cleaved S-2444 : pyroglutamyl-glycyl-arginine-p-nitroanilide S-2251 : D-valyl-leucyl-lysine-p-nitroanilide

D-Ile-Pro-Arg-CH₂Cl : D-isoleucyl-prolyl-arginine chloromethyl ketone IU : international units KIU : kallikrein inhibitor units

SDS : sodium dodecylsulphate

PAGE : polyacrylamide gel electrophoresis DTE : dithioerythritol DTT : dithiothreitol PEG : polyethylene glycol ELISA : enzyme-linked immunosorbent assay CHO cells : Chinese hamster ovary cells DHFR : dihydrofolate reductase BSA : bovine serum albumine dNTP : deoxyribonucleoside triphosphate

finger : NH₂-terminal region of t-PA, homologous to finger-like domains in fibronectin, which are involved in its affinity for fibrin kringle : triple loop disulfide bonded structures occurring in plasminogen (five),prothrombin (two), t-PA (two) and u-PA (one)

7

Table

**Amino acid composition of the t-PA/scu-PA hybrid**

| Amino acid | t-PA/scu-PA | sequence |
|---|---|---|
| Asx | 47 | 45 |
| Thr | 31 | 33 |
| Ser | 48 | 48 |
| Glx | 60 | 52 |
| Pro | 33 | 27 |
| Gly | 44 | 44 |
| Ala | 25 | 28 |
| Val | 16 | 22 |
| Met | 7 | 8 |
| Ile | 16 | 23 |
| Leu | 35 | 35 |
| Tyr | 25 | 27 |
| Phe | 16 | 16 |
| His | 14 | 15 |
| Lys | 27 | 28 |
| Arg | 33 | 32 |

The values represent means of duplicate analysis and are normalized to 483 which is the number of amino acids in the sequence, excluding Cys and Trp (which were not determined).

Legends

**Figure 1**

Nucleotide sequence and corresponding amion acid sequence decoding for the NH₂-terminal amino acids 1 through 263 of t-PA and the COOH-terminal amino acids 144 through 411 of scu-PA, respecitively.

**Figure 2**

Physical map of the different plasmids used in the deletion mutagenesis and the expression vector construction. Thin lines are bacterial sequences, the open boxes represent t-PA cDNA sequences, boxes indicated with + are scu-PA sequences, black boxes represent the SV40 early promotor enhancer region, hatched boxes the rabbit β-globin gene 3'-untranslated region, including a polyadenylation signal, and the stippled boxes represent the mouse DHFR cDNA. Restriction endonuclease sites indicated are: B : BglII, E : EcoRI, H : HindIII, S : Sau3AI, Ss : SstI.

Start and stop codons are overlined, and codons for the amino acid residues that are spliced together are indicated by the underlined amino acids. The plasmid sections that are underlined indicate restriction fragments used to construct M13t-PA-scu-PA, the sections that are indicated with an interrupted line, indicate fragments used to construct the expression plasmid PSVt-PAu-PADHFR.

**Figure 3** Treatment of t-PA scu-Pa with plasmin
Generation of urokinase-like amidolytic activity expressed in IU/mg following addition of plasmin to a final concentration of 30nM ( ● ), 60nM ( ■ ) or 90nM ( ▲ ) to 2,0μM t-PA scu-PA hybrid.

**Figure 4** Lineweaver-Burk plots of the activation of plasminogen by t-PA scu-PA (A) and by t-PA tcu-PA (B).

**Figure 5**
Effect of CNBr-digested fibrinogen on the activation of plasminogen by t-PA/tcu-PA hybrid (1,25nM). ( ● ): without; ( ▲ ): 0,25μM and ( ■ ): 0.50μM CNBr-digested fibrinogen.

**Figure 6** Binding to fibrin clots
Open symbols: residual antigen measured with u-PA ELISA; closed symbols: measured with t-PA ELISA. (o): scu-PA, (□): tcu-PA; (■): t-PA; (△,▲): t-PA/scu-PA hybrid; (▽, ▼): t-PA/tcu-PA hybrid. Concentrations of t-PA or u-PA related antigen were measured in the supernatant after thrombin-induced clotting of fibrinogen.

**Figure 7**
Lysis of $^{125}$I-fibrin labeled human plasma clots immersed in human plasma
A: scu-PA; B: tcu-PA; C: t-PA/scu-PA; D: t-PA/tcu-PA;
E: t-PA
scu-PA ( ■ : 8; ▼ : 16; ▲: 24; ●: 32; *: 48; ◆:64 IU/ml)
tcu-PA ( ■: 5; ▼ : 10; ▲: 20; ●: 30; *: 40 IU/ml)
t-PA/scu-PA ( ■: 3; ▼ : 6; ▲: 9; ●: 12; *: 18; ◆: 24 IU/ml)
t-PA/tcu-PA ( ■: 5; ▼ : 10; ▲: 15; ●: 20 IU/ml)
t-PA ( ■: 2; ▼ : 4; ▲: 8; ●: 16 IU/ml)

**Figure 8**
Influence of perincubation of plasminogen activators with plasma on their fibrinolytic properties
A: scu-PA; B: tcu-PA; C: t-PA/scu-PA; D: t-PA/tcu-PA
scu-PA ( ■: 0; ▼ : 16; ●: 32; *: 48; ◆: 64 IU/ml)
tcu-PA ( ▼ : 10; ▲: 20; ●: 30; *: 40 IU/ml)
t-PA/scu-PA ( ■: 0; ▼ : 6; ▲: 9; ●: 12; *: 18 IU/ml)
t-PA/tcu-PA ( ■: 5; ▼ : 10; ▲: 15; ●: 20 IU/ml)

## Claims

1. Plasminogen activators obtained by expression of fused cDNA encoding fragments of human tissue-type plasminogen activator (t-PA) and of human urokinase-type plasminogen activator (u-PA), <u>characterized by</u> combining the fibrin-affinity of t-PA with the fibrin-specificity of u-PA in the single chain form (scu-PA) and/or with the enzymatic activity of u-PA having the two-chain form (tcu-PA).

2. Plasminogen activator according to claim 1 having an improved fibrin-specificity of clot lysis in a plasma milieu.

3. Plasminogen activator according to claim 1 or 2 being a hybrid molecule obtained by fusion of at least a region part of the $NH_2$-terminal region of t-PA to at least a part region of the COOH-terminal region of scu-PA.

4. Plasminogen activator according to claim 3 composed of the $NH_2$-terminal amino acids 1 to 263 of t-PA, fused to the COOH-terminal amino acids 144 to 411 of scu-PA.

5. Plasminogen activator according to claim 4 having a peptide chain as illustrated in fig. 1.

6. A recombinant DNA molecule comprising the coding sequence, which yields a translation product having improved fibrin-specificity.

7. A recombinant DNA molecule according to claim 6 comprising the coding sequence of at least a region part of the $NH_2$-terminal region of t-PA fused in frame to the coding sequence of at least a region part of the COOH-terminal region of scu-PA, which yields a translation product having improved fibrin-specificity.

8. A recombinant molecule according to claim 7 encoding the $NH_2$-terminal 1 to 263 amino acids of t-PA and the COOH-terminal amino acids 144 to 411 of scu-PA, fused in frame.

9. A recombinant DNA molecule according to claim 7 containing the nucleotide sequence illustrated in fig. 1.

10. A mammalian expression vector containing the DNA sequence of claim 6-9.

11. A transfected mammalian cell line which comprises the recombinant DNA molecule of claim 10.

12. A method for the preparation of plasminogen activators according to claim 1-5, wherein the plasminogen activator is obtained from the cell culture medium or a cell extract of a cell line according to claim 11.

13. A method according to claim 12, wherein the plasminogen activator is isolated from the culture medium or from the extract by chromatography on Zinc-chelate-Sepharose, lysine-Sepharose, SP-Sephadex, gel filtration benzamidine-Sepharose, insolubilized polyclonal or monoclonal antibodies raised against t-PA or against u-PA, or combinations thereof.

14. Substance obtained by the expression of fused cDNA according to claim 1-5, for use as an active therapeutic substance.

15. Plasminogen activator according to claim 1-5 for use as a thrombolytic active substance.

16. Medical preparation comprising a substance obtained by expression of fused cDNA according to claim 1-5 and a pharmaceutically acceptable excipient.

17. Medical preparation with thrombolytic activity comprising a plasminogen activator according to claim 1-5 and a pharmaceutically acceptable inert excipient.

18. Method for the treatment of patients with thromboembolic disease with the characteristic that an intravenous infusion liquid is used, containing a pharmaceutical composition according to claim 16.

TTAAAGGAGGCCGGAGCTGTGGGGAGCTCGAGACTGAGATCCTACAGGAGTCCAGGGCTGGAGAGAAAACCTCTGCGAGGAAAGGGAAGGA

<div style="text-align:center">-35</div>

MetAspAlaMetLysArgGlyLeuCysCysValLeuLeuLeuCysGlyAlaVal
GCAAGCCGTGAATTTAAGGGACGCTGTGAAGCAATCATGGATGCAATGAAGAGAGGGCTCTGCTGTGTGCTGCTGCTGTGTGGAGCAGTC

<div style="text-align:center">1</div>

PheValSerProSerGlnGluIleHisAlaArgPheArgArgGlyAlaArgSerTyrGlnValIleCysArgAspGluLysThrGlnMet
TTCGTTTCGCCCAGCCAGGAAATCCATGCCCGATTCAGAAGAGGAGCCAGATCTTACCAAGTGATCTGCAGAGATGAAAAAACGCAGATG

IleTyrGlnGlnHisGlnSerTrpLeuArgProValLeuArgSerAsnArgValGluTyrCysTrpCysAsnSerGlyArgAlaGlnCys
ATATACCAGCAACATCAGTCATGGCTGCGCCCTGTGCTCAGAAGCAACCGGGTGGAATATTGCTGGTGCAACAGTGGCAGGGCACAGTGC

<div style="text-align:center">50</div>

HisSerValProValLysSerCysSerGluProArgCysPheAsnGlyGlyThrCysGlnGlnAlaLeuTyrPheSerAspPheValCys
CACTCAGTGCCTGTCAAAAGTTGCAGCGAGCCAAGGTGTTTCAACGGGGGCACCTGCCAGCAGGCCCTGTACTTCTCAGATTTCGTGTGC

<div style="text-align:center">100</div>

GlnCysProGluGlyPheAlaGlyLysCysCysGluIleAspThrArgAlaThrCysTyrGluAspGlnGlyIleSerTyrArgGlyThr
CAGTGCCCCGAAGGATTTGCTGGGAAGTGCTGTGAAATAGATACCAGGGCCACGTGCTACGAGGACCAGGGCATCAGCTACAGGGGCACG

TrpSerThrAlaGluSerGlyAlaGluCysThrAsnTrpAsnSerSerAlaLeuAlaGlnLysProTyrSerGlyArgArgProAspAla
TGGAGCACAGCGGAGAGTGGCGCCGAGTGCACCAACTGGAACAGCAGCGCGTTGGCCCAGAAGCCCTACAGCGGGCGGAGGCCAGACGCC

<div style="text-align:center">150</div>

IleArgLeuGlyLeuGlyAsnHisAsnTyrCysArgAsnProAspArgAspSerLysProTrpCysTyrValPheLysAlaGlyLysTyr
ATCAGGCTGGGCCTGGGGAACCACAACTACTGCAGAAACCCAGATCGAGACTCAAAGCCCTGGTGCTACGTCTTTAAGGCGGGGAAGTAC

SerSerGluPheCysSerThrProAlaCysSerGluGlyAsnSerAspCysTyrPheGlyAsnGlySerAlaTyrArgGlyThrHisSer
AGCTCAGAGTTCTGCAGCACCCCTGCCTGCTCTGAGGGAAACAGTGACTGCTACTTTGGGAATGGGTCAGCCTACCGTGGCACGCACAGC

FIG.1a  0 275 606

```
                    200
LeuThrGluSerGlyAlaSerCysLeuProTrpAsnSerMetIleLeuIleGlyLysValTyrThrAlaGlnAsnProSerAlaGlnAla
CTCACCGAGTCGGGTGCCTCCTGCCTCCCGTGGAATTCCATGATCCTGATAGGCAAGGTTTACACAGCACAGAACCCCAGTGCCCAGGCA          901

                                                                    250
LeuGlyLeuGlyLysHisAsnTyrCysArgAsnProAspGlyAspAlaLysProTrpCysHisValLeuLysAsnArgArgLeuThrTrp
CTGGGCCTGGGCAAACATAATTACTGCCGGAATCCTGATGGGGATGCCAAGCCCTGGTGCCACGTGCTGAAGAACCGCAGGCTGACGTGG          991

              263144                       150
GluTyrCysAspValProSerCysSerThrLeuLysPheGlnCysGlyGlnLysThrLeuArgProArgPheLysIleIleGlyGlyGlu
GAGTACTGTGATGTGCCCTCCTGCTCCACCTTAAAATTTCAGTGTGGCCAAAAGACTCTGAGGCCCCGCTTTAAGATTATTGGGGGAGAA         1081

PheThrThrIleGluAsnGlnProTrpPheAlaAlaIleTyrArgArgHisArgGlyGlySerValThrTyrValCysGlyGlySerLeu
TTCACCACCATCGAGAACCAGCCCTGGTTTGCGGCCATCTACAGGAGGCACCGGGGGGGCTCTGTCACCTACGTGTGTGGAGGCAGCCTC         1171

              200
MetSerProCysTrpValIleSerAlaThrHisCysPheIleAspTyrProLysLysGluAspTyrIleValTyrLeuGlyArgSerArg
ATGAGCCCTTGCTGGGTGATCAGCGCCACACACTGCTTCATTGATTACCCAAAGAAGGAGGACTACATCGTCTACCTGGGTCGCTCAAGG         1261

                                                            250
LeuAsnSerAsnThrGlnGlyGluMetLysPheGluValGluAsnLeuIleLeuHisLysAspTyrSerAlaAspThrLeuAlaHisHis
CTTAACTCCAACACGCAAGGGGAGATGAAGTTTGAGGTGGAAAACCTCATCCTACACAAGGACTACAGCGCTGACACGCTTGCTCACCAC         1351

AsnAspIleAlaLeuLeuLysIleArgSerLysGluGlyArgCysAlaGlnProSerArgThrIleGlnThrIleCysLeuProSerMet
AACGACATTGCCTTGCTGAAGATCCGTTCCAAGGAGGGCAGGTGTGCGCAGCCATCCCGGACTATACAGACCATCTGCCTGCCCTCGATG         1441

                    300
TyrAsnAspProGlnPheGlyThrSerCysGluIleThrGlyPheGlyLysGluAsnSerThrAspTyrLeuTyrProGluGlnLeuLys
TATAACGATCCCCAGTTTGGCACAAGCTGTGAGATCACTGGCTTTGGAAAAGAGAATTCTACCGACTATCTCTATCCGGAGCAGCTGAAA         1531

MetThrValValLysLeuIleSerHisArgGluCysGlnGlnProHisTyrTyrGlySerGluValThrThrLysMetLeuCysAlaAla
ATGACTGTTGTGAAGCTGATTTCCCACCGGGAGTGTCAGCAGCCCCACTACTACGGCTCTGAAGTCACCACCAAAATGCTATGTGCTGCT         1621
```

FIG.1b

0 275 606

                                350
AspProGlnTrpLysThrAspSerCysGlnGlyAspSerGlyGlyProLeuValCysSerLeuGlnGlyArgMetThrLeuThrGlyIle
GACCCCCAATGGAAAACAGATTCCTGCCAGGGAGACTCAGGGGGACCCCTCGTCTGTTCCCTCCAAGGCCGCATGACTTTGACTGGAATT      1711

                                                                            400
ValSerTrpGlyArgGlyCysAlaLeuLysAspLysProGlyValTyrThrArgValSerHisPheLeuProTrpIleArgSerHisThr
GTGAGCTGGGGCCGTGGATGTGCCCTGAAGGACAAGCCAGGCGTCTACACGAGAGTCTCACACTTCTTACCCTGGATCCGCAGTCACACC      1801

                    411
LysGluGluAsnGlyLeuAlaLeu
AAGGAAGAGAATGGCCTGGCCCTCTGAGGGTCCCCAGGGAGGAAACGGGCACCACCCGCTTTCTTGCTGGTTGTCATTTTTGCAGTAGAG      1891


TCATCTCCATCAGGAAGCTT      1911

FIG. 2

FIG. 3

FIG. 4a

# FIG. 4 b

FIG. 5

# FIG. 6

A

B

0 275 606

G    (mg/ml)

FIG. 7

0 275 606

# FIG. 8

0 275 606

European Patent Office

**PARTIAL EUROPEAN SEARCH REPORT**
which under Rule 45 of the European Patent Convention
shall be considered, for the purposes of subsequent
proceedings, as the European search report

Application number

EP 87 20 2610

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP - A - 0 155 387 (BEECHAM)<br><br>* Page 1, line 24 - page 2, line 28; page 4, lines 19-24; example 5; claims * | 1 | C 12 N 15/00<br>C 12 N 9/72<br>A 61 K 37/54 |
| Y | PROC.NATL.ACAD.SCI. USA, vol. 81, no. 17, September 1984, pages 5355-5359 Washington, DC, US; T. NY et al.: "The structure of the human tissue-type plasminogen activator gene; correlation of intron and exon structures to functional and structural domains"<br><br>* Page 5355, column 1, lines 1-47; page 5358, column 2, line 9 - page 5359, column 1, line 28 * | 1 | |
| D,A | BIOTECHNOLOGY, vol. 3, no. 10, October 1985, pages 923-929 US; W. HOLMES et al. : "Cloning and expression of the gene for pro-urekinase in Escherichia coli" | | |

TECHNICAL FIELDS
SEARCHED (Int. Cl.4)

C 12 N

## INCOMPLETE SEARCH                                    -2-

The Search Division considers that the present European patent application does not comply with the provisions of the European Patent Convention to such an extent that it is not possible to carry out a meaningful search into the state of the art on the basis of some of the claims.

Claims searched completely:

Claims searched incompletely:

Claims not searched:          18

Reason for the limitation of the search:

Method for treatment of the human or animal body by surgery or therapy (see art. 52(4) of the European Patent Convention).

| Place of search | Date of completion of the search | Examiner |
|---|---|---|
| THE HAGUE | 19-04-1988 | DELANGHE |

| Category | Citation of document with indication, where appropriate, of relevant passages | Relevant to claim |
|---|---|---|
| | * Abstract; introduction; discussion * | 1 |
| A | EP - A - 0 093 619 (GENENTECH) | |
| | * Page 9, line 19 - page 10, line 34; page 21, lines 1-6 * | 1 |
| P,X | THE JOURNAL OF BIOLOGICAL CHEMISTRY, vol. 262, no. 22, 5th August 1987, pages 10855-10862 US; L. NELLES et al.: "Characterization of a fusion protein consisting of amino acids 1 to 263 of tissue-type plasminogen activator and amino acids 144 to 411 of urokinase-type plasminogen activator" | |
| | * Whole document * | 1-17 |
| P,X | THE JOURNAL OF BIOLOGICAL CHEMISTRY, vol. 262, no. 24, 25th August 18987, pages 11.711 - 11.778, US; L. PIERARD et al.: "Mutant and chimeric recombinant plasminogen activators" | |
| | * Whole document * | 1-3, 6,7 |
| P,X | EP - A - 0 231 883 (SAGAMI) | |
| | * Claims; figure 3 * | 1-3, 6,7 |

DOCUMENTS CONSIDERED TO BE RELEVANT

CLASSIFICATION OF THE APPLICATION (Int. Cl.4)

TECHNICAL FIELDS SEARCHED (Int Cl.4)

--------------------

EPO Form 1505.3   06.78